# EUROPÄISCHE PATENTSCHRIFT

(11) **EP 3 623 037 B1**
(45) Veröffentlichungstag und Bekanntmachung des Hinweises auf die Patenterteilung: **27.10.2021**
(21) Anmeldenummer: 18000728.8
(22) Anmeldetag: 12.09.2018
(51) Int. Cl.: B01D 61/20, B01D 61/30, B01D 63/02, A61M 1/16

(54) **SYSTEM AUS FILTER UND FILTERHALTER MIT FIXIERVORRICHTUNG**
FILTER SYSTEM WITH FILTER AND FILTER HOLDER COMPRISING A FIXATION SYSTEM
SYSTÈME DE FILTRAGE AVEC UN FILTRE ET UN PORTE-FILTRE COMPRENANT UN SYSTÈME DE FIXATION

(43) Veröffentlichungstag der Anmeldung: 18.03.2020
(73) Patentinhaber: Völker, Manfred, 63825 Blankenbach (DE)
(72) Erfinder: Völker, Manfred, 63825 Blankenbach (DE); Schmitt, Cornelia, 63877 Sailauf (DE); Thomas, Reinhart, 63843 Niedernberg (DE)
(74) Vertreter: Flosdorff, Jürgen

(56) Entgegenhaltungen:
- EP-A1- 0 887 100
- WO-A1-03/028859
- DE-C1- 19 925 297
- JP-A- 2015 177 973
- JP-A- 2016 049 177

## Beschreibung

Die Erfindung betrifft ein Filtersystem gemäß dem Oberbegriff des Anspruchs 1.

Gegenstand der Erfindung ist die kostengünstige Steril- oder Ultrafiltration von Flüssigkeiten, um einen höheren Reinheitsgrad insbesondere bei bakteriellen Verschmutzungen zu erhalten. Dabei kann ein modifizierter Kapillarfilter verwendet, werden, wie er beispielsweise in der Dialyse eingesetzt wird. Ausführung und Bauart sind nicht auf Dialysatoren begrenzt, sondern je nach Anforderung z.B. an geforderte Filtrationsmengen, sind auch andere z.B. größere Bauformen und Filtermedien möglich.

Hauptsächlich soll der Filter bei der Trinkwasseraufbereitung, aber auch z. B. nach Austauschern oder nach durch Membranfiltration aufbereiteten Wässern eingesetzt werden.

Das Einsatzgebiet erstreckt sich damit neben der Trinkwasseraufbereitung, über Laborwasser-, Pharmawasser-, med. techn. Wasser für Spüllösungsaufbereitungen bis zu Anwendungen im Krankenhaus.

Filter dieser Art sind nicht neu, allerdings sind diese Filter kostenintensive Verbrauchmaterialien. Die große Versorgungsbasis durch Dialysatoren und deren exzellente Qualität hinsichtlich bakterieller Rückhaltungen, sowie die zunehmende Kontamination des Wassers sind eine Grundlage dieser Erfindung.

Die EP 0 887 100 A1 offenbart eine Filtervorrichtung der Eingangs genannten Art. Bei dieser Filtervorrichtung werden die Anschlusskonnektoren des Filterhalters durch Federkraft vorgeschoben, wobei sie in die Anschlüsse des Filters eintreten, mit der Folge, dass der Strömungsquerschnitt in den hydraulischen Anschlüssen des Filters verringert ist. Die Verbindung zwischen den Anschlüssen des Filters und den Anschlusskonnektoren des Filterhalters wird dabei durch Federkraft hervorgerufen. Diese Ausbildung hat den Nachteil, dass die hydraulische Verbindung zwischen dem Filter und dem Filterhalter undicht werden kann.

Nachteile des Standes der Technik sind insbesondere die mangelhafte Druckfestigkeit von Filter der oben beschriebenen Art, sowie das Fehlen einer einfachen, fehlersicheren und druckfesten Konnektion für ungeübte Anwender in den oben beschrieben Einsatzbereichen. Dabei sollen die gegebenen Strömungsquerschnitte der Anschlüsse nicht beeinträchtigt, d.h. nicht minimiert bzw. verkleinert werden.

Die zu lösende Aufgabe ist daher einen kostengünstigen, druckfesten Filter ohne strömungs-minimierende Anschlüsse zu entwickeln, der sicher und einfach zu konnektieren bzw. auszutauschen ist.

Dazu erhält der Dialysator druckfeste Kappen mit je einem hydraulischen Anschluss Konnektor. Die Anschluss-Konnektoren der beiden Kappen verlaufen vertikal in Linie mit den am Dialysator Gehäuse angespritzten hydraulischen Anschlüssen. Der so modifizierte Filter wird an einen noch zu beschreibenden Filterhalter konnektiert. Diese bilden zusammen das Filtrationssystem. Der Filterhalter selbst kann stationär unter Tisch-, oder an Wasseraufbereitungsgeräten, oder als zusätzlich Filtrations-Entnahmeeinheit im Labor z.B. auch an einer Wand befestigt oder eingehängt werden.

Hauptfunktionseinheiten des Filtrationssystems sind die Elemente einer Verschlussmechanik im Filterhalter, die eine sichere hydraulische Verbindung zwischen Filter und Filterhalter herstellt, sowie die Elemente einer mechanischen Filterfixierung im Filter und Filterhalter, die sowohl die fehlerfreie Anordnung des Filters in dem Filterhalter-, als auch zunächst die mechanische Verbindung und damit auch die formschlüssige hydraulische Verbindung zwischen Filter und Filterhalter ermöglicht.

Zur mechanischen Verbindung wird der Filter, der an beiden Kappen T-förmig ausgebildete Fixierungen besitzt, in die Nuten der Fixierung am Filterhalters bis zum Anschlag eingeschoben.

Zur Erleichterung und zielgerichteten Einbringen des Filters sind die Fixierungen am Filterhalter so ausgebildet, dass auch ungeübte Anwender den Filterwechsel ausführen können. Dazu dienen nach oben verbreiternde V-förmige Nuten und eine etwas längere untere Filterfixierung am Filterhalter, um den Filter zunächst einseitig, am unteren Ende aufstecken zu können.

Die beiden Fixierungen am Filterhalter sind Bestandteil der jeweils oberen und unteren Verschlussmechanik.

Durch Drehen am Konnektionshebel der unteren- als auch oberen Verschlussmechanik wird die hydraulische Verbindung hergestellt.

Dazu sind auf einer Drehwelle des Konnektionshebels jeweils zwei Druckstücke verdrehsicher angebracht. Diese Druckstücke pressen die hydraulischen Verbindungskonnektoren an ihre jeweiligen Gegenstücke des Filters.

Die Druckstücke oben und unten der Verschlussmechanik sind in ihrer Endlage zweifach positioniert und stehen in der Raststellung etwas über 9 Uhr und können sich aufgrund des Drehwinkels und der Fixierungen nicht selbstständig zurückstellen. Fixiert werden die Druckstücke zum einen durch Führung in einem Spalt der seitlich führt und den Drehwinkel über 9 Uhr hinaus begrenzt, als auch durch eine mechanisch Raststellung die durch Federdruck unterstützt wird.

Um maßliche Abweichungen insbesondere der Abstandsmaße oder Winkelmaße der vier vertikal angeordneten Anschluss Konnektoren des Filters auszugleichen, sind zwei der vier Gegenstücke, die zwei mittleren Anschluss-Konnektoren am Filterhalter durch einen umlaufenden Ringspalt spielend gelagert. Es könne jedoch auch alle vier Konnektoren des Filterhalterhalters spielend gelagert werden.

Alle Gegen Konnektoren am Filtergehäuse dichten umfangsseitig ihre Konnektoren am Filter ab, damit liegen keine Querschnittsminderungen der hydraulischen Verbindungen vor.

Zum Öffnen werden die Konnektionshebel der oberen und unteren Verschlussmechanik zurückgedreht, dabei werden die Anschluss-Konnektoren am Filterhalter durch Federkraft zurückgeführt und der Filter kann aus der Fixierung entnommen werden.

Damit ist im Wesentlichen die eingangs beschriebene Aufgabe erfüllt.

Um den Austritt von Flüssigkeiten zu vermeiden besteht die Möglichkeit in das Gehäuse des Filterhalterhalters Ventile mit einzubringen. Diese Ausführung wird in den nachfolgenden Beschreibungen der Figuren dargestellt.

Da die Filtrationsleistung dieser Filter begrenzt ist, besteht die Möglichkeit durch Hinzunahme einer Mengen- oder Flussüberwachung und deren vielfältigen Ausgestaltungen hinsichtlich Signalübertragung, eine Meldung oder Trenddarstellung an Technik oder Anwender weiterzugeben. Zur Vergrößerung der Filtrationsleistung können Filtrationssysteme dieser Art auch parallel und seriell verbunden werden.

### Beschreibung des Filtersystems

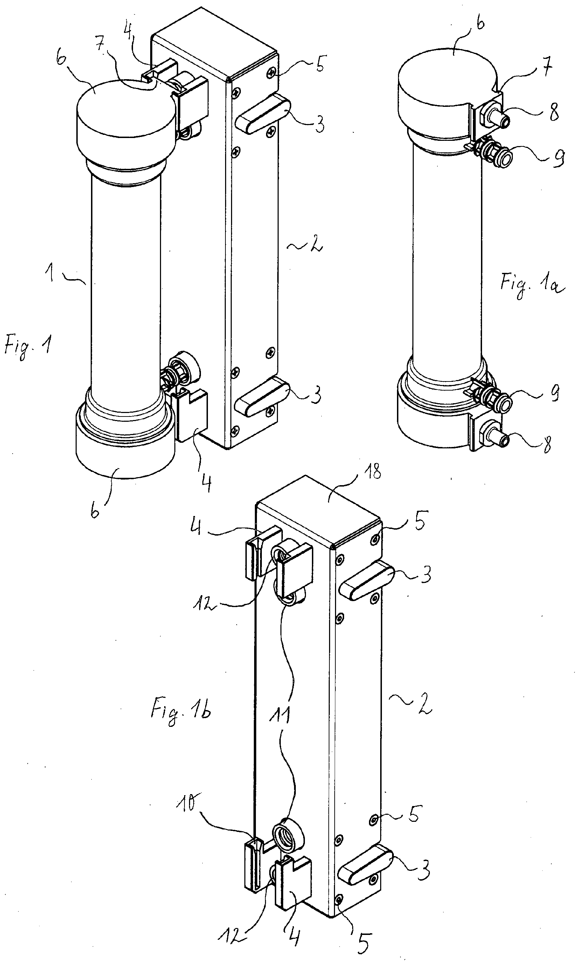

**Figur 1** zeigt ein Filtersystem bestehend aus Hohlfaserfilter 1 und dem Filterhalter 2. Der Hohlfaserfilter 1 ist mit den beiden Filterkappen 6 verschraubt. Die Filterkappen sind seitlich T-förmig so ausgeführte, das der Filter in die äußere Filterfixierung 4 des Filterhalters 2 eingehängt werden kann. Seitlich am Filterhalter 2 befinden sich Konnektionshebel3, die sowohl links- als auch rechtsseitig am Filterhalter ***2*** angebracht werden können, sodass drei Einbaulagen , links, rechts, mittig des Filterhalters 2, möglich sind.
**Figur 1** **a** zeigt die an der Filterkappe befindlichen T-förmigen Fixierhilfen mit denen der Filter mechanisch in die äußere Filterfixierung ***4*** des Filterhalters eingehängt wird. Am Filter selbst befinden sich zwei hydraulische Anschlüsse ***9***. An den Filterkappen ***6*** sind ebenfalls hydraulische Anschlüsse ***8*** angebracht. Je nach Filterbauform und Größe können die Anschlüsse 9 ebenfalls in den jeweilige untere bzw. obere Filterkappen 6 mit angeordnet sein.
**Figur 1b** zeigt den Filterhalter ***2*** mit den beiden Konnektionshebel ***3*** mit der die später zu beschreibende Filterkonnektion bzw. die Verschlussmechanik bedient wird. Am Filterhalter selbst sind die äußeren Filterfixierungen ***4*** mit ihrer trichterförmig erweiterten Nut ***10*** angebracht, in die die Fixierhilfe ***7*** des Filters eingeschoben wird. Die untere äußere Filterfixierung besitzt eine L-förmige Verlängerung nach oben, um das Einbringen des Filters ***1*** zu erleichtern. Ebenfalls geht aus der Zeichnung die Lage der Anschlusskonnektoren ***11*** und ***12*** hervor.

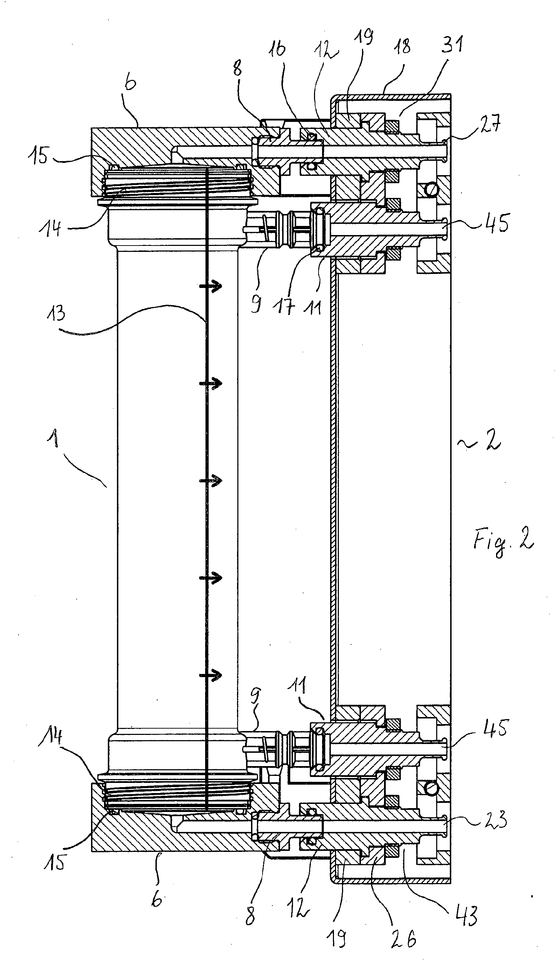

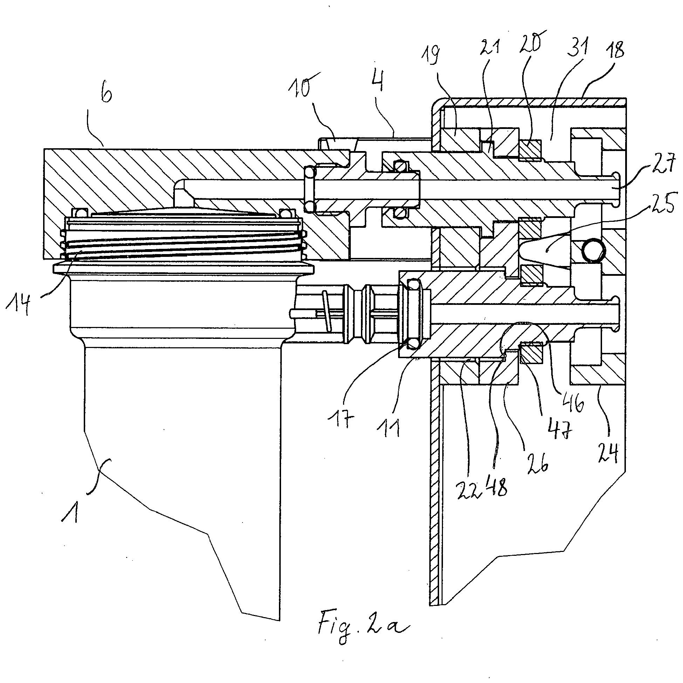

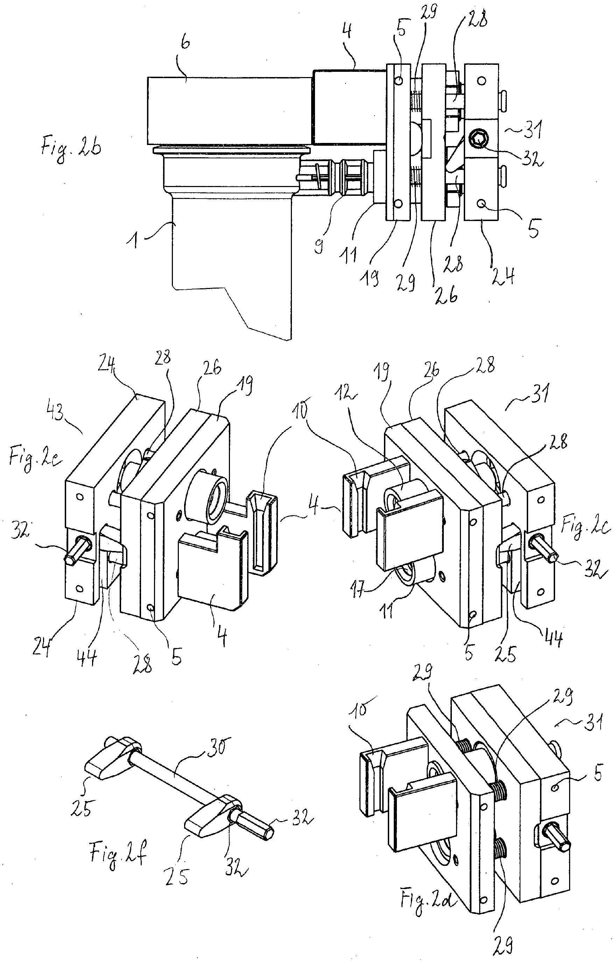

**Figur 2** zeigt die hydraulische Verbindung des Filtersystems im Schnitt.
   Z.B. wird über Anschluss ***23***, die hier als Schlauchtülle ausgebildet ist, Flüssigkeiten an die Filtermembran/ Kapillare ***13*** heran geführt. Die hier gezeigte Ausführungsform der Anschlüsse 23, 45, 27 sind auch durch Gewinde Anschlüsse ersetzbar.
   Bei einer Dead End Filtration wird der Anschluss ***27*** verschlossen, oder Anschluss 27 wird als Entlüftungs-, Zirkulations- oder Retentatabfluss genutzt.
   Das Filtrat wird an den Anschlüsse ***45*** entnommen. Je nach Filtrationserfordernissen sind Primär- und Sekundärseite des Filters 1 umtauschbar, sodass die vorher beschriebene Funktion der Anschlüsse umgekehrt wird.
   Die Filterkappen ***6*** sind mittels Gewinde ***14*** auf den Filter ***1*** so eingeschraubt, dass die Anschlüsse ***8*** und ***9*** vertikal fluchtend sind. Die Filterkappen ***6*** sind mit Dichtring ***15*** stirnseitig gegen die Umgebung abgedichtet. Zur zusätzlichen Verdrehsicherung des Gewindes 14 besteht die nicht dargestellte Möglichkeit einer Verrastung in der Form eines Umfangseitigen Steges der in einer Nut einrastet. Nut und Stege sind wahlwiese am Filter oder Kappe ausführführbar.
   Im Filterhaltergehäuse ***18*** befindet sich die obere Verschlussmechanik ***31*** und die untere Verschlussmechanik ***43***, mit diesen die Anschlusskonnektoren ***11*** und ***12*** in die jeweiligen Gegenstücke des Filters ***8*** und ***9*** dichtend herangeführt werden.
**Figur 2** **a** zeigt beispielsweise die obere Verschlussmechanik ***31*** im konnektiertem Zustand mit dem Filter ***1***.
   Die Verschlussmechanik ***31*/*43*** ist dreiteilig ausgeführt und besteht aus einer Gegenlager- und Führungsplatte ***24*** aus der Konnektormitnehmerplatte ***26*** und einer vorderen Fixierplatte ***19*.**
   Mit der Konnektormitnehmerplatte ***26*** sind die Konnektoren 12,11 mechanisch verbunden. Konnektor 12 sitzt mit einem Flansch in einer Kreistasche 46 u. wird gegenseitig mit einer Mutter 20 fixiert, Konnektor 11 sitzt ebenfalls in einer Kreistasche 46 der Mitnehmerplatte allerdings wird die Fixierung der Mutter 20 durch einen schrägen Anschlag 47 begrenzt und zusätzlich durch Ringspalt 22 kann Konnektor 11 locker Toleranzen ausgleichen. Die Toleranzausgleichende Bauform und Einbaulage ist auch für Konnektor 12 möglich.
   Fig. 2a zeigt die Lage des Druckstücks ***25*** so, dass die Konnektorenmitnehmplatte ***26***, mit Konnektorenanschlüsse ***11*** und ***12*** in Verschluss mit den Gegenkonnektoren des Filters ***8*** und ***9*** gebracht sind.
Figur 2b zeigt die die Verschlussmechanik ***31*** in mittlerer Stellung.
   Die dreiteilige Verschlussmechanik 31,43 wird durch vier Führungsbolzen ***28*** auf Abstand gehalten. Auf diesen Führungsbolzen gleitet die Konnektormitnehmerplatte 26 während des Bewegungsvorgangs. Die Konnektorenmitnehmerplatte wird mittels Druckstück ***25*** in ihre Anschlussposition gebracht und nach zurückdrehen des Druckstückes 25, über die Rückstellfeder ***29*** in ihrer Ausgangslage zurückgedrückt.
   Die Lage der Verschlussmechanik 31, 43 im Filterhalter 2 wird durch die Befestigung 5 im Filterhaltergehäuse 18 bestimmt.
**Figur 2** **c** zeigt die obere Verschlussmechanik ***31*** im konnektierten Zustand, dabei sind die Anschlusskonnektoren ***11*** und ***12*** nach vorne geschoben. Die Konnektormitnehmerplatte ***26*** liegt an der vorderen Fixierplatte ***19*** an, das Druckstück ***25*** ist uhrzeigermäßig betrachtet etwas über 09.00 Uhr angehoben. Dabei liegt das leicht abgerundete vordere Ende des Druckstücks 25 in einer hier nicht dargestellten Mulde der Konnektormitnehmerplatte ***26*** . Die nahezu auf Block gesetzten vier Federn 29 jeder Verschlussmechnik drücken Platte ***26*** gegen das Druckstück 25 und sorgen hier für Formschluss.
   Die Federn ***29*** können hierbei in nicht dargestellte ringförmige Auskreisungen der Platte ***19*/*26*** eintauchen. Die Druckstücke ***25*** drücken beidseits der Platte 26 und sind in der Führungsnut ***44*** so gehalten das sowohl ein Überdrehen nach oben als auch ein seitliches Verschieben nicht möglich ist.
**Figur 2d** zeigt die Verschlussmechanik ***31*** in entspannter Form, dabei drücken die vier Federn 29 die Konnektormitnehmerplatte 26 in ihre Endlage.
**Figur 2** **e** zeigt die untere Verschlussmechanik ***43*** mit der nach oben trichterförmig verlängerten Fixierhilfe ***4*** in geschlossenem Zustand.
**Figur 2f** zeigt die Welle ***30*** auf der die Druckstücke ***25*** verdrehsicher aufgeschoben sind, hier wurde beispielsweise als Verdrehsicherung ein Sechskant gewählt, jede andere Form der Verdrehsicherung zum Beispiel Vierkant, ist ebenso möglich.
   Der äußere Anschluss für den Konnektionshebel ***3*** ist ebenfalls mit einer Verdrehsicherung ***32*** ausgeführt. Der Konnektionshebel ***3*** hat einen nach innen führenden Bund mit einer kreisförmigen Nut in das ein Ausschnitt des Gehäuses 18 eingreift. Ein Abnehmen der Konnektionshebel 3 bei montierter Verschlussmechanik ***31*/*43*** ist daher nicht möglich ist.

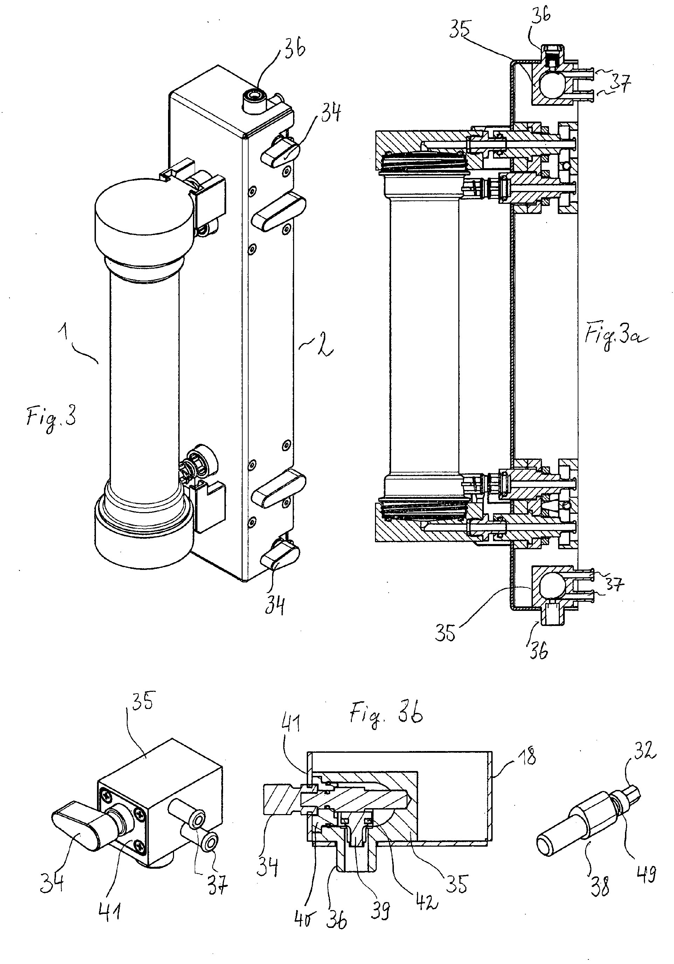

**Figur 3** zeigt einen Filterhalter mit dem Verschlusshebel ***34*** und gewindeförmigen Anschlüssen 36 mit denen das Filtersystem an eine Haus- oder Trinkwasserinstallation angeschlossen werden kann.
**Figur 3** **a** zeigt beispielsweise die Verschlusseinheit ***35*** in Schnitt ohne Ventilkörper. Verschlusseinheit 35 kann erforderlich werden um einen Wasseraustritt bei Filterwechsel zu vermeiden.
**Figur 3b** zeigt die Verschlusseinheit ***35*** im Schnitt. Über Anschluss ***36*** kann zu- oder abführend Flüssigkeit angeschlossen werden, mittels Verschlusshebel ***34*** wird die exzenterförmige Welle ***38*** so gedreht, dass der Ventilstößel ***39*** mit der Dichtung ***42*** den Wasserein- respektive Ausgang verschließt. Die Anschlüsse ***37*** sind jeweils parallel in eine weitere Verschlauchung zu vereinfachen.

| | |
|---|---|
| 1. | Hohlfaserfilter |
| 2. | Filterhalter |
| 3. | Konnektionshebel |
| 4. | Äußere Filterfixierung |
| 5. | Befestigung Konnektions-Mechanik |
| 6. | Filterkappen |
| 7. | Fixierhilfe |
| 8. | Filter Anschluss Filterkappe |
| 9. | Filter Anschluss Gehäuse |
| 10. | Filteraufnahmenut mit trichterförmiger Öffnung |
| 11. | Anschlusskonnektor Filtergehäuse |
| 12. | Anschlusskonnektor Filterkappe |
| 13. | Filterkapillare/ Membrane |
| 14. | Filtergewinde |
| 15. | Kappendichtung |
| 16. | Konnektordichtung |
| 17. | Konnektordichtung |
| 18. | Filterhaltergehäuse |
| 19. | Vordere-Fixierplatte mit Rückstellfedern und Dämpfer |
| 20. | Befestigungs - Mutter Anschlusskonnektoren |
| 21. | Haltebundklemmung |
| 22. | Umlaufender Ringspalt |
| 23. | Anschluss.z.B. Wasserzuführung |
| 24. | Gegenlager u. Führungsplatte- Druckstück |
| 25. | Druckstück |
| 26. | Konnektormitnehmer-Platte |
| 27. | Anschluss |
| 28. | Führungsbolzen |
| 29. | Rückstellfedern |
| 30. | Drehwelle für Druckstück |
| 31. | Obere Verschlussmechanik |
| 32. | Verdrehsicherung Druckstück |
| 33. | Konnektionshebelhaltenut |
| 34. | Verschlusshebel |
| 35. | Verschlusseinheit |
| 36. | Anschluss |
| 37. | Anschlüsse |
| 38. | Excenterventil |
| 39. | Ventilstößel mit Dichtung |
| 40. | Dichtbuchse |
| 41. | Klemmblech |
| 42. | Dichtung |
| 43. | Untere Verschlussmechanik |
| 44. | Führungsnut Druckstück |
| 45. | Anschluss z.B Filtrat |
| 46. | Kreistasche |
| 47. | Anschlag Mutter |
| 48. | Schräger Anschlag Anschlusskonnektor |
| 49. | Dichtungsnut |
| 50. | |

## Patentansprüche

1. Filtersystem, das einen Filter und einen Filterhalter aufweist,
wobei der Filter (1) mit einer oberen und einer unteren Filterkappe (6) verbunden ist,
wobei an dem Umfang der Filterkappen (6) je eine querschnittlich T-förmige Fixierhilfe (7) mit seitlich überstehendem Quersteg angebracht ist, die vertikal miteinander fluchten,
wobei an einer Außenseite des Gehäuses (18) des Filterhalters (2) zwei Filterfixierungen (4) angebracht sind mit Nuten, in die die Querstege einhängbar sind,
wobei von dem Filter (1) und/oder den Filterkappen (6) vier hydraulische Anschlüsse (8, 9) seitlich vorstehen, die vertikal miteinander fluchten,
wobei der Filterhalter (2) vier Anschlusskonnektoren (11, 12) aufweist, die durch eine obere und eine untere Verschlussmechanik (31, 43) vorschiebbar sind, **dadurch gekennzeichnet, dass**
die vier Anschlusskonnektoren (11, 12) gegen Federkraft vorschiebbar sind, bis sie in einer Endstellung einrasten, in der die Anschlusskonnektoren (11, 12) dicht mit den hydraulischen Anschlüssen (8, 9) verbunden sind, und dass die Anschlusskonnektoren (11, 12) in der Endstellung die Anschlüsse (8, 9) umgreifen, so dass deren Strömungsquerschnitt nicht verringert ist.

2. Filtersystem nach Anspruch 1,
**dadurch gekennzeichnet,**
**dass** der Filter (1) zwei hydraulische Anschlüsse (9) aufweist, und
**dass** jede Filterkappe (6) einen hydraulischen Anschluss (8) hat.

3. Filtersystem nach einem der Ansprüche 1 bis 2,
**dadurch gekennzeichnet,**
**dass** jede Verschlussmechanik (31, 43) eine hintere Gegenlager- und Führungsplatte (24), eine vordere Fixierplatte (19) und dazwischen eine verschiebliche Konnektormitnehmerplatte (26) aufweist und
**dass** die Anschlusskonnektoren (12, 11) mechanisch mit der Konnektormitnehmerplatte (26) verbunden sind,
**dass** zwischen der vorderen Fixierplatte (19) und der Konnektormitnehmerplatte (26) Rückstellfedern (29) angeordnet sind, die die Konnektormitnehmerplatte (26) von der vorderen Fixierplatte (19) wegdrücken,
**dass** die Gegenlager- und Führungsplatte (24) mit wenigstens einem Druckstück (25) versehen ist, das auf einer Drehwelle (30) sitzt, durch deren Drehung das wenigstens eine Druckstück (25) die Konnektormitnehmerplatte (26) gegen Federkraft zur vorderen Fixierplatte (19) vorschiebt, so dass die Konnektorenanschlüsse (11, 12) dicht an den Konnektoren (8, 9) anliegen, wobei das wenigstens eine Druckstück (25) in der Endstellung einrastet, und dass seitlich am Filterhalter (2) Konnektionshebel (3) angeordnet sind, die zur Betätigung der Druckstücke (25) mit der Drehwelle (30) drehfest verbunden sind.

4. Filtersystem nach Anspruch 3,
dadurchgekennzeichnet, dass die Konnektormitnehmerplatte (26) verschieblich auf Führungsbolzen (28) sitzt.

5. Filtersystem nach einem der Ansprüche 1 bis 4,
**dadurch gekennzeichnet,**
**dass** wenigstens einer der Anschlusskonnektoren (11 oder 12) von einem Ringspalt (22) umgeben ist, so dass Toleranzen ausgleichbar sind.

## Claims

1. A filter system, which includes a filter and a filter holder, wherein the filter (1) is connected to an upper and a lower filter cap (6), wherein attached to the periphery of the filter caps (6) there are respective fixing aids (7) of T-shape in cross-section with a laterally projecting transverse web, which are vertically aligned with one another, wherein attached to an outer surface of the housing (18) of the filter holder (2) there are two filter fixings (4) with grooves, in which the transverse webs may be mounted, wherein projecting laterally from the filter (1) and/or the filter caps (6) there are four hydraulic connections (8, 9), which are vertically aligned with one another, wherein the filter holder (2) includes four connectors (11, 12), which may be advanced by an upper and a lower fastening mechanism (31, 43), **characterised in that** the four connectors (11, 12) may be advanced against spring force until they lock in an end position, in which the connectors (11, 12) are sealingly connected to the hydraulic connections (8, 9), and that the connectors (11, 12) engage around the connections (8, 9) in the end position so that their flow cross-section is not reduced.

2. A filter system as claimed in Claim 1, **characterised in that** the filter (1) includes two hydraulic connections (9) and that each filter cap (6) has a hydraulic connection (8).

3. A filter system as claimed in one of Claims 1 to 2, **characterised in that** each fastening mechanism (31, 43) includes a rear counter bearing and guide plate (24), a front fixing plate (19) and a movable connector drive plate (26) between them and that the connectors (11, 12) are mechanically connected to the connector drive plate (26), that arranged between the front fixing plate (19) and the connector drive plate (26) there are return springs (29), which urge the connector drive plate (26) away from the front fixing plate (19), that the counter bearing and guide plate (24) is provided with at least one pressure member (25), which is located on a rotary shaft (30), by rotation of which the at least one pressure member (25) advances the connector drive plate (26) against spring force to the front fixing plate (19) so that the connectors (11, 12) sealingly engage the connectors (8, 9), wherein the at least one pressure member (25) locks in the end position and that arranged laterally on the filter holder (2) there are connection levers (3), which are rotationally fixedly connected to the rotary shaft (30) for actuation of the pressure members (25).

4. A filter system as claimed in Claim 3, **characterised in that** the connector drive plate (26) is movably located on guide pegs (28).

5. A filter system as claimed in one of Claims 1 to 4, **characterised in that** at least one of the connectors (11 or 12) is surrounded by an annular gap (22) so that tolerances may be compensated for.

## Revendications

1. Système de filtrage, qui présente un filtre et un support de filtre,
dans lequel le filtre (1) est relié à un couvercle filtrant supérieur et un couvercle filtrant inférieur (6),
dans lequel est installé sur la périphérie des couvercles filtrants (6) respectivement un aide à la fixation (7) en forme de T dans la section transversale avec une entretoise transversale faisant saillie latéralement, les aides de fixation étant en affleurement verticalement les unes avec les autres,
dans lequel sont installées sur un côté extérieur du boîtier (18) du support de filtre (2) deux fixations de filtre (4) avec des rainures, dans lesquelles les entretoises transversales peuvent être accrochées,
dans lequel font saillie latéralement du filtre (1) et/ou des couvercles filtrants (6) quatre raccords (8, 9) hydrauliques, qui sont en affleurement verticalement les uns avec les autres,
dans lequel le support de filtre (2) présente quatre connecteurs de raccordement (11, 12), qui peuvent être avancés par coulissement par un mécanisme de fermeture supérieure et un mécanisme de fermeture inférieur (31, 43),
**caractérisé en ce que**
les quatre connecteurs de raccordement (11, 12) peuvent être avancés par glissement à l'encontre d'une force de ressort, jusqu'à ce qu'ils s'enclenchent dans une position finale, dans laquelle les connecteurs de raccordement (11, 12) sont reliés de manière étanche aux raccords hydrauliques (8, 9),
et
que les connecteurs de raccordement (11, 12) entourent dans la position finale les raccordements (8, 9) de sorte que leur section transversale d'écoulement n'est pas réduite.

2. Système filtrant selon la revendication 1,
**caractérisé en ce**
**que** le filtre (1) présente deux raccordements hydrauliques (9), et
**que** chaque couvercle filtrant (6) a un raccordement hydraulique (8).

3. Système filtrant selon l'une quelconque des revendications 1 à 2,
**caractérisé en ce**
**que** chaque mécanisme de fermeture (31, 43) présente une plaque de contre-palier arrière et une plaque de guidage (24), une plaque de fixation avant (19) et, de manière intercalée, une plaque d'entraîneur de connecteur (26) pouvant coulisser, et
**que** les connecteurs de raccordement (12, 11) sont reliés de manière mécanique à la plaque d'entraîneur de connecteur (26),
**que** sont disposés entre la plaque de fixation avant (19) et la plaque d'entraîneur de connecteur (26) des ressorts de rappel (29), qui éloignent en la poussant la plaque d'entraîneur de connecteur (26) de la plaque de fixation avant (19),
**que** la plaque de contre-palier et la plaque de guidage (24) sont pourvues d'au moins une pièce de pression (25), qui siège sur un arbre de rotation (30), par la rotation duquel l'au moins une pièce de pression (25) avance par coulissement la plaque d'entraîneur de connecteur (26) à l'encontre de la force de ressort par rapport à la plaque de fixation avant (19) de sorte que les raccordements de connecteur (11, 12) reposent de manière étanche sur les connecteurs (8, 9), dans lequel l'au moins une pièce de pression (25) s'enclenche dans la position finale, et que sont disposés latéralement sur le support de filtre (2) des leviers de connexion (3), qui sont reliés de manière solidaire en rotation à l'arbre de rotation (30) pour l'actionnement des pièces de pression (25).

4. Système filtrant selon la revendication 3,
**caractérisé en ce que** la plaque d'entraîneur de connecteur (26) siège de manière à pouvoir coulisser sur des boulons de guidage (28).

5. Système filtrant selon l'une quelconque des revendications 1 à 4,
**caractérisé en ce**
**qu'**au moins un des connecteurs de raccordement (11 ou 12) est entouré par une fente annulaire (22) de sorte que des tolérances peuvent être compensées.
